(19) Europäisches Patentamt
European Patent Office
Office européen des brevets

(11) **EP 1 227 080 B1**

(12) **EUROPÄISCHE PATENTSCHRIFT**

(45) Veröffentlichungstag und Bekanntmachung des Hinweises auf die Patenterteilung:
**29.09.2004   Patentblatt 2004/40**

(51) Int Cl.$^7$: **C07C 249/04**, C07C 251/44

(21) Anmeldenummer: **01128780.2**

(22) Anmeldetag: **04.12.2001**

(54) **Durch Ammoniumsalze oder substituierte Ammoniumsalze cokatalysierstes Verfahren zur Herstellung von Oximen**

Process for the preparation von oximes cocatalysed by ammonium salts or substituted ammonium salts

Procédé de préparation d'oximes cocatalysé par des sels d'ammonium ou d'ammonium substitués

(84) Benannte Vertragsstaaten:
**AT BE CH CY DE DK ES FI FR GB GR IE IT LI LU MC NL PT SE TR**

(30) Priorität: **26.01.2001  DE 10103581**

(43) Veröffentlichungstag der Anmeldung:
**31.07.2002   Patentblatt 2002/31**

(73) Patentinhaber: **Degussa AG**
**40474 Düsseldorf (DE)**

(72) Erfinder:
• **Thiele, Georg Friedrich, Dr.**
**63450 Hanau (DE)**
• **Schiffer, Thomas, Dr.**
**45721 Haltern (DE)**
• **Oenbrink, Georg, Dr.**
**48249 Dülmen (DE)**

(56) Entgegenhaltungen:
**EP-A- 0 690 045**          **JP-A- 2000 072 738**

**Beschreibung**

Die vorliegende Erfindung betrifft ein katalytisches Verfahren zur Herstellung von Oximen durch Umsetzung von Aldehyden oder Ketonen in flüssiger Phase mit Wasserstoffperoxid und Ammoniak in Gegenwart heterogener Katalysatoren, die auf Basis von Titan, Silizium und Sauerstoff aufgebaut sind, und von Ammoniumsalzen oder substituierten Ammoniumsalzen.

In den europäischen Patentanmeldungen EP-A-0 208 311, EP-A-0 267 362 und EP-A-0 299 430 sowie in der amerikanischen Patentschrift US 4 794 198 wird die Darstellung und Aktivierung eines Katalysators auf Basis von Titan, Silizium und Sauerstoff sowie dessen Verwendung zur Synthese von Oximen ausgehend von Aldehyden oder Ketonen wie beispielsweise Cyclohexanon durch Umsetzung mit Wasserstoffperoxid und Ammoniak beschrieben (sogenannte Ammoximation). Die Katalysatoren weisen üblicherweise ein Verhältnis Silizium : Titan größer 30 auf. Ein typischer Vertreter ist der Titansilikalit TS 1.

Während die Synthese kleiner aliphatischer und cycloaliphatischer Oxime ausgehend von Ketonen mit bis zu 6 Kohlenstoffatomen wie zum Beispiel Cyclopentanon und Cyclohexanon gute Ergebnisse an zahlreichen, gemäß oben genannten Schriften dargestellten und aktivierten Titansilikaliten als Katalysator liefert, fallen die Resultate bei größeren oder sterisch anspruchsvolleren Carbonylverbindungen wie zum Beispiel Acetophenon oder Cyclododecanon deutlich schlechter aus. Insbesondere sind die Reaktionsgeschwindigkeit, der Umsatz der eingesetzten Carbonylverbindung und die Ausbeute bezüglich Wasserstoffperoxid (zur Ammoximation verwendetes $H_2O_2$ : Gesamtmenge benötigtes $H_2O_2 \cdot 100\ \%$) bei diesen Versuchen unbefriedigend.

Werden bei Cyclohexanon in den Beispielen der europäischen Patentanmeldung EP-A-0 267 362 Umsätze von über 90 % bei einem Peroxidverlust von unter 10 % erzielt (Beispiele 22 und 24), so werden bei vergleichbaren Reaktionsbedingungen mit Acetophenon nur noch Umsätze von 50,8 % bei einem Peroxidverlust von 48,9 % erreicht. Die Umsetzung von Cyclododecanon wird in der genannten Anmeldung beansprucht, jedoch kein konkretes Beispiel zu Umsatz und Peroxidverlust angegeben.

Die deutlich schlechteren Ergebnisse bei großen oder sterisch anspruchsvollen Carbonylverbindungen lassen sich unter anderem darauf zurückführen, dass große Carbonylverbindungen wie beispielsweise Cyclododecanon (CDON) nicht oder nur langsam durch die Poren des Titansilikalit-Katalysators dringen können. Dadurch kann es zu einer räumlichen Trennung der Teilschritte Hydroxylaminbildung (1) und Oximierung des Ketons (2) (in den Reaktionsgleichungen dargestellt am Beispiel des Cyclododecanons (CDON)), kommen.

$$(1) \qquad NH_3 + H_2O_2 \rightarrow H_2O + NH_2OH$$

$$(2) \qquad NH_2OH + CDON \rightarrow CDON\text{-}Oxim + H_2O$$

$$(3) \qquad 2\,NH_2OH + H_2O_2 \rightarrow 4\,H_2O + N_2$$

$$(4) \qquad 2\,H_2O_2 \rightarrow 2\,H_2O + O_2$$

Wird das gebildete Hydroxylamin nicht sofort, beziehungsweise nicht vollständig mit der jeweiligen Carbonylverbindung umgesetzt, so treten verstärkt verschiedene Nebenreaktionen auf, zum Beispiel die Weiteroxidation des Hydroxylamins mit vorhandenem Wasserstoffperoxid, formal dargestellt als stöchiometrische Gleichung (3).

Zur Vollständigkeit sei auch auf eine weitere, die Peroxidselektivität betreffende Nebenreaktion hingewiesen, nämlich die basen- oder metallkatalysierte Zersetzung des Wasserstoffperoxids gemäß Gleichung (4).

Enichem beansprucht in der deutschen Patentanmeldung DE 195 21 011 A1 (entspricht US 5 498 793) ein amorphes Siliziumdioxid als Cokatalysator zur Ammoximation von Acetophenon und Cyclododecanon. Durch Zugabe von amorphem Siliziumdioxid lässt sich danach der Umsatz bei Cyclododecanon nach 8 Stunden Reaktionszeit auf 85,5 % beziehungsweise 85,2 % steigern (DE 195 21 011 A1, Beispiele 5 und 6) im Vergleich zu 76,6 % ohne Cokatalysator. Die Peroxidausbeute steigt gleichzeitig von 65,8 % auf 71,4 % beziehungsweise 72,3 % an. Das von Enichem beschriebene Verfahren führt zu einer leichten Verbesserung von Umsatz und Peroxidausbeute, doch weist die beschriebene Reaktionsführung mehrere Nachteile auf, die einen technischen Einsatz unwirtschaftlich erscheinen lassen:

Die Menge an Katalysator und an Cokatalysator bezogen auf das eingesetzte Keton ist in den Beispielen mit je bis zu 25 Gew.-% bei den Versuchen mit Cyclododecanon sehr hoch.

Trotz der hohen Katalysatorkonzentration ist die Umsatzrate gering und die Reaktion langsam. Auch nach einer Gesamtreaktionszeit von 8 Stunden liegt die Oxim-Ausbeute noch weit von einem vollständigen Umsatz entfernt.

Nebenreaktionen gemäß den Teilschritten (3) und (4) senken die Raum-Zeit-Ausbeute der Ammoximation, vor allem wirken sie sich negativ auf die Peroxid-Selektivität aus. Die Peroxidselektivität liegt bei der Ammoximation von CDON ohne Cokatalysator zum Teil bei unter 50 %, durch diverse Cokatalysatoren kann sie bei CDON gemäß der deutschen Patentanmeldung P 100 47 435.7 auf etwa 60 % - 70 % angehoben werden. Für ein technisches Verfahren ist dies allerdings noch nicht zufriedenstellend.

In der EP-A 690 045 wird ein Verfahren zur Ammonoxidation von Cyclohexanon offenbart, bei dem Ammoniumhydroxid, also eine wässrige Lösung an Ammoniak, eingesetzt wird.

Es bestand daher die Aufgabe, ein Verfahren zu finden, bei dem die Ammoximation insbesondere größerer und sterisch anspruchsvollerer Carbonylverbindungen dahingehend verbessert wird, dass bei möglichst vollständigem Umsatz an Carbonylverbindungen und hoher Raum-Zeit-Ausbeute die Peroxidselektivität deutlich verbessert wird.

Überraschend wurde gefunden, dass die Lösung der Aufgabe dann gelingt, wenn während der Reaktion Ammoniumsalze oder substituierte Ammoniumsalze anwesend sind gemäß Anspruch 1.

Die vorliegende Erfindung betrifft daher ein Verfahren zur Herstellung von Oximen durch Umsetzung von Carbonylverbindungen, insbesondere von Aldehyden oder Ketonen, wie zum Beispiel Acetophenon, und cyclischen Ketonen mit 7 bis 20 Kohlenstoffatomen, wie zum Beispiel Cyclododecanon, in flüssiger Phase mit Wasserstoffperoxid und Ammoniak in Gegenwart eines oder mehrerer heterogener Katalysatoren, dem optional weitere Komponenten wie beispielsweise saure oder neutrale anorganische oder organische Feststoffe als Cokatalysator und/oder Binder zugesetzt werden können, und von Ammoniumsalzen oder substituierten Ammoniumsalzen, wobei die Ammoniumkonzentration in der Reaktionsmischung mindestens 100 ppm beträgt, berechnet als $[NR_1R_2R_3R_4]^{\oplus}$.

Der heterogene Katalysator ist auf Basis von Titan, Silizium und Sauerstoff aufgebaut. Der Katalysator ist vorzugsweise ein sogenannter Titansilikalit. Bevorzugt besitzen die Katalysatoren eine mikro- oder mesoporöse Struktur. Sie weisen üblicherweise ein Verhältnis Silizium : Titan größer 30 auf. Ein typischer und besonders aktiverVertreter ist der Titansilikalit TS1.

Neben dem Titansilikalit-Katalysator können optional weitere Verbindungen als Cokatalysator anwesend sein, die an der Oberfläche oder in Poren lewis- und/oder brönstedsaure Zentren aufweisen. Als nicht limitierende Beispiele für anorganische Cokatalysatoren seien zum Beispiel Aluminiumoxide, insbesondere saure Aluminiumoxide, saure aktivierte Alumosilikate wie zum Beispiel Bentonit, Montmorrillonit und Kaolinit gemäß der deutschen Patentanmeldung P 100 47 435.7 oder amorphes Siliziumdioxid gemäß der deutschen Patentanmeldung DE 195 21 011 A1 (Enichem), genannt. Als nicht limitierende Beispiele für Cokatalysatoren, die auf organische Trägermaterialien aufgebaut sind, seien saure und stark saure Ionenaustauscherharze wie Amberlyst 15 oder Nafion NR 50 gemäß der deutschen Patentanmeldung P 100 47 435.7 genannt.

Katalysator und Cokatalysator können jeweils als Feststoff sowohl als Pulver als auch als Formkörper verwendet werden. Das Gewichtsverhältnis zwischen Katalysator (vorzugsweise der genannte Titansilikalit) und Cokatalysator variiert üblicherweise zwischen 0,1 : 1 und 10 : 1. Bevorzugt übernimmt beim Formkörper der feste Cokatalysator auch die Funktion des Binders.

Erfindungsgemäß liegen die als Cokatalysator wirkenden Ammoniumionen oder substituierten Ammoniumionen, im Folgenden der Einfachheit halber als Ammoniumionen bezeichnet, homogen gelöst in der flüssigen Phase vor. Dabei sind folgende Möglichkeiten zum Einbringen der Ammoniumionen geeignet:

- die Ammoniumionen werden in Form eines geeigneten Ammoniumsalzes im Lösemittel gelöst. Bei diskontinuierlicher Fahrweise werden sie bevorzugt mit dem Titan-Katalystor im Reaktor vorgelegt. Bei kontinuierlicher Fahrweise werden sie laufend mit der Carbonylverbindung in den Reaktor eingebracht.
- die Ammoniumionen werden erst im Reaktor gebildet, indem zur Reaktionsmischung eine geeignete Säure zugegeben wird, welche mit dem im Reaktor vorhandenen Ammoniak oder einem Amin sofort zu den entsprechenden Ammoniumsalzen neutralisiert wird. Anstelle der freien Säure kann genauso gut ein geeignetes Derivat davon (zum Beispiel ein Anhydrid) zugegeben werden, welches mit Ammoniak beziehungsweise einem Amin Ammoniumionen beziehungsweise substituierte Ammoniumionen bildet.

Die Ammonium- oder substituierten Ammoniumsalze gehorchen der allgemeinen Formel $[NR_1R_2R_3R_4]^{\oplus}X^{\ominus}$, wobei $R_1$, $R_2$, $R_3$ und $R_4$ unabhängig voneinander Wasserstoff, einen aliphatischen, unverzweigten oder verzweigten Alkylrest mit 1 bis 20, vorzugsweise 1 bis 6 Kohlenstoffatomen wie z. B. den Methyl-, Ethyl-, Propyl- oder Butyl-Rest oder einen cycloaliphatischen Rest mit 3 bis 12 Kohlenstoffatomen oder einen aromatischen Rest mit insgesamt 6 bis 12 Kohlenstoffatomen und $X^{\ominus}$ ein Anion einer Säure, welches so gewählt wird, dass die entsprechende Ammoniumverbindung in dem System ausreichend löslich ist, bedeutet und sind dabei homogen gelöst im Reaktionssystem anwesend, wobei die Ammoniumkonzentration in der Reaktionsmischung mindestens 100 ppm beträgt, berechnet als $[NR_1R_2R_3R_4]^{\oplus}$, wobei $X^{\ominus}$ ein Halogenid-, Nitrat- oder Phosphation oder ein Anion einer organischen Carbonsäure ist. Die Alkylketten bei den substituierten Ammoniumverbindungen sind geeignet, um auch in weniger polaren Lösemitteln eine ausreichende Löslichkeit zu gewährleisten.

Wie die Beispiele 1 - 4 und 6 zeigen, werden die Ammoniumionen nicht stöchiometrisch, sondern in katalytischen Mengen benötigt.

In der Regel liegt in der Reaktionsmischung eine sehr geringe Konzentration an Ammoniumionen latent vor. Sie ergibt sich aus der Tatsache, dass handelsübliches Wasserstoffperoxid meist durch Ortho- oder Pyrophosphorsäure stabilisiert wird. Bei Anwesenheit von Ammoniak bilden sich durch Neutralisation Ammoniumionen in geringen Konzentrationen aus.

Die japanische Patentanmeldung JP 98-247281 (Mitsubishi Gas Chemical Co.) beansprucht bei der Ammoximation einen Gehalt an Peroxidstabilisatoren von 1 - 1000 ppm bezogen auf die Menge an Wasserstoffperoxid bei der Ammoximierung.

Im zitierten Beispiel des englischen Abstracts von JP 98-247281 wird Wasserstoffperoxid mit 30 ppm Orthophosphorsäure bezogen auf $H_2O_2$ eingesetzt. Ein entsprechendes Wasserstoffperoxid mit ebenfalls 30 ppm Orthophosphorsäure als Stabilisator wurde auch in den folgenden Beispielen 1 - 4 und 6 sowie in Vergleichsbeispielen 5 und 7 der vorliegenden Anmeldung verwendet.

Unter der Annahme, dass die Phosphorsäure mit drei Äquivalenten Ammoniak vollständig zu Ammoniumphosphat $(NH_4)_3PO_4$ umgesetzt wird, ergibt sich bei den Beispielen 1 - 7 in der Reaktionsmischung eine Ammoniumkonzentration von maximal 0,4 ppm verursacht durch die vorhandene Phosphorsäure.

Wird der in JP 98-247281 beanspruchte Maximalwert von 1000 ppm Phosphorsäure bezogen auf $H_2O_2$ als Stabilisator für das Wasserstoffperoxid verwendet, ergäbe sich bei der in den Beispielen angegebenen Fahrweise ein Gehalt an Ammoniumionen von maximal 14 ppm in der Reaktionsmischung.

Bei den Experimenten zeigte sich deutlich, dass die durch einen Peroxidstabilisator eingebrachten Konzentrationen an Ammoniumionen zur Katalyse der Ammoximierung nicht ausreichen, sondern zusätzlich Ammoniumionen durch eines der oben beschriebenen Verfahren in die Reaktionsmischung eingebracht werden müssen. Zudem ist es für hohe Umsätze und Peroxidselektivitäten wichtig, dass eine ausreichend hohe Ammoniumionenkonzentration schon zu Beginn der Reaktion vorliegt, sich nicht erst im Laufe der Reaktion durch Zugabe mit $H_2O_2$ einstellt.

Für einen merklichen katalytischen Effekt wird eine Ammoniumkonzentration in der Reaktionsmischung von mindestens 100 ppm, bevorzugt mindestens 250 ppm und ganz besonders bevorzugt mindestens 500 ppm benötigt, jeweils berechnet als $(NR_1R_2R_3R_4)^\oplus$.

Die Obergrenze bei der Ammoniumionenkonzentration ergibt sich zum einen durch die Löslichkeit des Salzes im jeweiligen Lösemittel, zum anderen dadurch, dass ab einer gewissen Höhe der Ammoniumionenkonzentration keine weitere Verbesserung von Umsatzrate und Peroxidselektivität mehr erzielt wird. Je nach Salz und Lösemittel kann sie bis zu 20 Gew.-% betragen.

Eingesetzt werden Ammoniumsalze, die im Lösemittel mäßig bis gut löslich sind. Neben den Halogeniden, dem Nitrat und dem Phosphat sind dies vor allem die Ammoniumsalze organischer Carbonsäuren und deren Mischungen. Als nicht limitierende Beispiele für bevorzugte Salze organischer Carbonsäuren seien Ammoniumformiat, Ammoniumacetat, Ammoniumpropionat, Ammoniumoxalat und Ammoniummalonat genannt.

Als Lösemittel dienen Verbindungen, die gegenüber Wasserstoffperoxid und Ammoniak stabil sind und sowohl für die Carbonylfunktion beziehungsweise das gebildete Oxim als auch für das verwendete Ammoniumsalz eine ausreichende Löslichkeit aufweisen. Vorzugsweise eignen sich als Lösemittel kurzkettige aliphatische, mit Wasser mischbare oder teilweise mischbare Alkohole wie zum Beispiel Methanol, Ethanol, n-Propanol, n-Butanol, Isobutanol, tert. -Butanol und tert.-Amylalkohol. Besonders geeignet sind für die Reaktion mit CDON Methanol, Ethanol und tert.-Butanol.

Ammoniak wird bei der Ammoximation als Reagenz benötigt und liegt im Überschuss vor. Er wird als konzentrierte, vorzugsweise wässrige Lösung (≥ 20 Gew.-%) oder bevorzugt als trockenes Gas eingeleitet. Durch den Überschuss an Ammoniak verläuft die Reaktion im basischen Milieu.

Wasserstoffperoxid wird als 0,1 bis 85gew.-%ige, vorzugsweise wässrige, Lösung eingesetzt. Bevorzugt werden konzentrierte wässrige Lösungen mit ≥ 30 Gew.-%.

Ziel bei der Auswahl von Ammoniak und Wasserstoffperoxid ist es jeweils, den Anteil Wasser im Lösemittel gering zu halten, da Wasser die Löslichkeit der Carbonylverbindungen, insbesondere der Oxime, senkt.

Die erfindungsgemäße Reaktion verläuft bezüglich der Ammoximation der Carbonylverbindung hochgradig selektiv. Bei Cyclododecanon wird bei vollständigen Umsätzen (>99,5 %) eine Selektivität des Oxims laut Gaschromatogramm (GC) von über 99 % erreicht. Wird zum Beispiel technisch reines Cyclododecanon eingesetzt, lassen sich als Nebenprodukte im GC lediglich Spuren von Cyclododecan und Cyclododecanol nachweisen, welche bereits im Cyclododecanon als Verunreinigung vorhanden waren. Bei Wassergehalten im Lösemittel von unter 1 % konnte Cyclododecanonimin als Zwischen-/Nebenprodukt in Spuren nachgewiesen werden, welches während der Reaktion reversibel zum Keton zurückreagiert und dann zum Oxim umgesetzt wird.

Die Reaktionstemperatur der Ammoximation liegt zwischen 20 °C und 150 °C, bevorzugt zwischen 50 °C und 120 °C und besonders bevorzugt zwischen 60 °C und 100 °C.

Der Reaktor wird dabei entweder bei Normaldruck, das heißt dem Dampfdruck des jeweiligen Lösemittels bei der gewählten Reaktionstemperatur, oder bei einem leichten Überdruck, vorzugsweise zwischen 1 bar und 10 bar betrieben. Der Überdruck kann mit Ammoniak oder einem Inertgas eingestellt werden. Wird der Reaktor verschlossen, steigt der Druck durch Bildung von gasförmigen Zersetzungsprodukten in Nebenreaktionen (vor allem Stickstoff) während der Reaktion an. Vorteilhaft ist es, den Reaktor isobar zu fahren, indem gasförmige Zersetzungsprodukte über einen leichten Abgasstrom mit Blasenzähler kontrolliert entweichen können und gegebenenfalls verbrauchter Ammoniak nachgedrückt wird.

Bei der Ammoximationsreaktion können Carbonylverbindung und Wasserstoffperoxid jeweils diskontinuierlich oder kontinuierlich zudosiert werden. Für einen vollständigen Alkanal/Alkanon-Umsatz wird ein leichter Überschuss an Peroxidlösung benötigt, welcher durch geeignete Reaktionsführung und die erfindungsgemäße Ammoniumionenkonzentration des Katalysatorsystems minimiert werden kann. Bei den Versuchen hat es sich als vorteilhaft erwiesen, zu Beginn der Reaktion entweder die Carbonylverbindung vorzulegen oder sie in äquimolaren Mengen parallel zum Wasserstoffperoxid zu dosieren und den benötigten Überschuss an Peroxid nach beendeter Carbonylzugabe gemäß dem Verbrauch nachzudosieren.

Um exakt gleiche Reaktionsbedingungen zu gewähren, wurde bei allen Beispielen jeweils frischer Katalysator (Titansilikalit TS1, Degussa-Hüls AG) der selben Charge eingesetzt. Es wurde für alle Versuche eine wässrige Lösung von Wasserstoffperoxid (50 Gew.-% mit 30 ppm Phosphorsäure als Stabilisator) verwendet. Eine zusätzliche Aktivierung des Katalysators vor der Reaktion erfolgte nicht. Der Katalysator wurde nach der Reaktion bei 75 °C über einen Druckfilter abgetrennt und konnte so mit hoher Restaktivität zurückgewonnen werden.

Beispiel 1:

In einem mit Stickstoff gespülten, beheizbaren 1,6 1-Druckreaktor aus Glas (Büchi-Autoklav) mit Magnetkupplung, Gaseinleitungsrührer (500 U/Min) und Druckregler wurden 5,25 g (68 mmol) Ammoniumacetat gelöst in 300 ml (243 g) Ethanol vorgelegt. 5,0 g Katalysator (TS1, Degussa-Hüls) wurden darin suspendiert. Bei 40 °C wurden 62,7 g (344 mmol) Cyclododecanon (CDON) in 251 g Ethanol zudosiert. Der Reaktor wurde auf 80 °C erwärmt und auf 0,1 bar entspannt, anschließend wurde langsam Ammoniakgas bis auf einen Druck von 1,6 bar aufgedrückt. Dabei wurden circa 13 g (ca. 765 mmol) Ammoniak zugegeben. Dies entspricht etwa 2,2 Äquimalenten bezogen auf Cyclododecanon.

Während der Reaktion wurde der Druck über einen leichten Abgasstrom konstant gehalten, mit entwichenes Ammoniakgas wurde nachdosiert (- 0,4 g/h). Über einen Zeitraum von 60 Minuten wurden 24,5 ml (28,56 g) (0,41 ml/min) einer 50gew.-%igen Wasserstoffperoxid-Lösung (entspricht 430 mmol (14,62 g) $H_2O_2$) über eine Pumpe zudosiert. Nach beendeter Peroxidzugabe ließ man die Reaktionsmischung noch 60 Minuten nachreagieren.

Der CDON-Umsatz wurde während der Reaktion per Gaschromatographie verfolgt und Wasserstoffperoxid iodometrisch bestimmt. Nach 120 Minuten betrug der CDON-Umsatz 99,95 %, es wurden 426 mmol $H_2O_2$ verbraucht, dies entspricht einer Peroxidselektivität von 80,6 %, bezogen auf umgesetztes CDON.

Weitere Ergebnisse sind in den Tabellen 1 - 3 zusammengefasst.

Beispiel 2:

Der Versuch wurde entsprechend Beispiel 1 durchgeführt.
Über einen Zeitraum von 60 Minuten wurden 22,0 ml (0,37 ml/min) einer 50gew.-%igen Wasserstoffperoxid-Lösung (entspricht 385 mmol (13,09 g) $H_2O_2$) über eine Pumpe zudosiert.

Nach beendeter Peroxidzugabe ließ man die Reaktionsmischung noch 60 Minuten nachreagieren.

Nach 120 Minuten betrug der CDON-Umsatz 99,90 %, es wurden 384 mmol $H_2O_2$ verbraucht, dies entspricht einer Peroxidselektivität von 89,2 %. Weitere Ergebnisse sind in den Tabellen 1 - 3 zusammengefasst.

Beispiel 3:

Der Versuch wurde entsprechend Beispiel 2 mit 2,63 g Ammoniumacetat wiederholt.
Über einen Zeitraum von 60 Minuten wurden 22,0 ml (0,37 ml/min) einer 50gew.-%igen Wasserstoffperoxid-Lösung (entspricht 385 mmol (13,09 g) $H_2O_2$) über eine Pumpe zudosiert. Nach 120 Minuten betrug der CDON-Umsatz 99,91 %, es wurden 382 mmol $H_2O_2$ verbraucht, dies entspricht einer Peroxidselektivität von 90,0 %. Weitere Ergebnisse sind in den Tabellen 1 - 3 zusammengefasst.

Beispiel 4:

Der Versuch wurde entsprechend Beispiel 2 mit 0,53 g Ammoniumacetat wiederholt.
Über einen Zeitraum von 60 Minuten wurden 22,0 ml (0,37 ml/min) einer 50gew.-%igen Wasserstoffperoxid-Lösung (entspricht 385 mmol (13,09 g) $H_2O_2$) über eine Pumpe zudosiert. Nach 120 Minuten betrug der CDON-Umsatz 79,42 %, es wurden 371 mmol $H_2O_2$ verbraucht, dies entspricht einer Peroxidselektivität von 73,5 %. Weitere Ergebnisse sind in den Tabellen 1 - 3 zusammengefasst.

Beispiel 5 (Vergleichsbeispiel):

Der Versuch wurde entsprechend den Beispielen 2 und 3, jedoch ohne Ammoniumacetat wiederholt.

Über einen Zeitraum von 60 Minuten wurden 22,0 ml (0,37 ml/min) einer 50gew.-%igen Wasserstoffperoxid-Lösung (entspricht 385 mmol (13,09 g) $H_2O_2$) über eine Pumpe zudosiert. Nach beendeter Peroxidzugabe ließ man die Reaktionsmischung noch 120 Minuten nachreagieren.

Der Umsatz wurde während der Reaktion per Gaschromatographie verfolgt und Wasserstoffperoxid iodometrisch bestimmt. Nach 120 Minuten betrug der CDON-Umsatz 32,80 %, nach 180 Minuten 33,31 %, es wurden 378 mmol $H_2O_2$ verbraucht, dies entspricht einer Peroxidselektivität von 30,3 %. Weitere Ergebnisse sind in den Tabellen 1 - 3 zusammengefasst.

Tabelle 1:

| Umsatz CDON über die Reaktionszeit | | | | |
|---|---|---|---|---|
| Beispiel Nr. (Salz) | 30 Min [%] | 60 Min [%] | 120 Min [%J | 180 Min [%] |
| 1 (5,25 g NH$_4$OAc) | 58,51 | 99,42 | 99,95 | - |
| 2 (5,25 g NH$_4$OAc) | 51,04 | 98,34 | 99,90 | - |
| 3 (2,63 g NH$_4$OAc) | 60,26 | 90,10 | 99,91 | - |
| 4 (0,53 g NH$_4$OAc) | 42,50 | 74,50 | 79,42 | - |
| 5 (ohne) | 12,10 | 28,85 | 32,80 | 33,31 |

Tabelle 2:

| Umsatzgeschwindigkeit | |
|---|---|
| Beispiel Nr. (Salz) | Umsatzrate nach 60 Min [mg Oxim/ (g TS1 *Min)] |
| 1 (5,25 g NH$_4$OAc) | 207,8 |
| 2 (5,25 g NH$_4$OAc) | 205,5 |
| 3 (2,63 g NH$_4$OAc) | 188,3 |
| 4 (0,53 g NH$_4$OAc) | 155,7 |
| 5 (ohne) | 60,3 |

Tabelle 3:

| umgesetztes Peroxid / Peroxidselektivität (%) | | |
|---|---|---|
| Beispiel Nr. (Salz) | 120 (180*) min verbrauchtes H$_2$O$_2$ [mmol] | Selektivität H$_2$O$_2$ bzw. umgesetztes CDON |
| 1 (5,25g NH$_4$OAc) | 426 | 80,6 % |
| 2 (5,25g NH$_4$OAc) | 384 | 89,2 % |
| 3 (2,63g NH$_4$OAc) | 382 | 90,0 % |
| 4 (0,53g NH$_4$OAc) | 371 | 73,5 % |
| 5 (ohne) * | 378 | 30,3 % |

Beispiele 6 und 7 zeigen die Umsetzung mit Acetophenon. Dabei wurden die für CDON optimierten Parametereinstellungen übernommen.

Beispiel 6:

Der Versuch wurde entsprechend Beispiel 1 wiederholt.
Anstelle von Cyclododecanon wurden 41,33 g (344 mmol) Acetophenon eingesetzt. Über einen Zeitraum von 60 Minuten wurden 24,5 ml (0,41 ml/min) einer 50gew.-%igen Wasserstoffperoxid-Lösung (entspricht 430 mmol (14,62 g) $H_2O_2$) über eine Pumpe zudosiert. Nach beendeter Peroxidzugabe ließ man die Reaktionsmischung noch 60 Minuten nachreagieren.

Nach 120 Minuten betrug der Acetophenon-Umsatz 84,29 %, es wurden 416 mmol $H_2O_2$ verbraucht, dies entspricht einer Peroxidselektivität von 69,7 %.

Beispiel 7 (Vergleichsbeispiel):

Der Versuch wurde entsprechend Beispiel 6 wiederholt, jedoch ohne den Zusatz von Ammoniumacetat. Über einen Zeitraum von 60 Minuten wurden 24,5 ml (0,41 ml/min) einer 50gew.-%igen wässrigen Wasserstoffperoxidlösung mit 30 ppm Orthophosphorsäure als Stabilisator (entspricht 430 mmol (14,62 g) $H_2O_2$) über eine Pumpe zudosiert. Nach beendeter Peroxidzugabe ließ man die Reaktionsmischung noch 60 Minuten nachreagieren.

Nach 120 Minuten betrug der Acetophenon-Umsatz 13,6 %; es wurden 406 mmol $H_2O_2$ verbraucht, dies entspricht einer Peroxidselektivität von 11,5 %.

Beispiel 8:

Der Versuchsaufbau aus Beispiel 1 wurde um einen Festbettreaktor mit Umwälzpumpe (600 ml/min) ergänzt. Als Festbettkatalysator wurden 50 g Formkörper (Granulat 1 mm Durchmesser) verwendet, welcher im Extruder hergestellt wurde aus 80 Gew.-% Titansilikalit TS1 (Degussa-Hüls AG) und 20 Gew.-% Aluminiumoxid Pural SB als saurer Cokatalysator. Die Reaktion erfolgte analog zu Beispiel 1 bei konstantem Druck (1,6 bar) und 80 °C.

608 g einer 12 gew.-%igen Lösungen von Cyclododecanon (73 g, 400 mmol) in Ethanol wurden vorgelegt und darin 5,8 g Ammoniumacetat gelöst. Dies entspricht einer Ammoniumionenkonzentration von 0,1 mol/l in der Reaktionsmischung.

Innerhalb von 3 Stunden wurden 25,1 ml (0,139 ml/min) einer 50 gew.-%igen Wasserstoffperoxidlösung (entspricht 440 mmol (14,96 g) $H_2O_2$) zugegeben. Nach beendeter Peroxidzugabe ließ man die Reaktion noch 1 Stunde nachreagieren. Nach 240 Minuten betrug der CDON-Umsatz 97,15 %. Es wurden 435 mmol $H_2O_2$ verbraucht. Dies entspricht einer Peroxidselektivität von 89,3 %.

**Patentansprüche**

1. Verfahren zur Herstellung von Oximen durch Umsetzung von Carbonylverbindungen in flüssiger Phase mit Wasserstoffperoxid und Ammoniak in Gegenwart eines heterogenen Katalysators, der auf Basis von Titan, Silizium und Sauerstoff aufgebaut ist,
   **dadurch gekennzeichnet,**
   **dass** Ammoniumsalze oder substituierte Ammoniumsalze der allgemeinen Formel $[NR_1R_2R_3R_4]^\oplus X^\ominus$, wobei $R_1$, $R_2$, $R_3$ und $R_4$ unabhängig voneinander Wasserstoff, einen aliphatischen, unverzweigten oder verzweigten Alkylrest mit 1 bis 20 Kohlenstoffatomen, einen cycloaliphatischen Rest mit 3 bis 12 Kohlenstoffatomen oder einen aromatischen Rest mit insgesamt 6 bis 12 Kohlenstoffatomen und $X^\ominus$ ein Anion einer Säure bedeutet, homogen gelöst im Reaktionssystem anwesend sind, wobei die Ammoniumkonzentration in der Reaktionsmischung mindestens 100 ppm beträgt, berechnet als $[NR_1R_2R_3R_4]^\oplus$, wobei $X^\ominus$ ein Halogenid-, Nitrat- oder Phosphation oder ein Anion einer organischen Carbonsäure ist.

2. Verfahren nach Anspruch 1,
   **dadurch gekennzeichnet,**
   **dass** die Konzentration der Ammoniumionen oder substituierten Ammoniumionen 100 ppm bis 20 Gew.-% in der Reaktionsmischung beträgt, wobei die Ammoniumverbindung oder substituierte Ammoniumverbindung homogen gelöst ist.

3. Verfahren nach Anspruch 1,
   **dadurch gekennzeichnet,**
   **dass** die Konzentration der Ammoniumionen oder substituierten Ammoniumionen 250 ppm bis 20 Gew.-% in der Reaktionsmischung beträgt, wobei die Ammoniumverbindung oder substituierte Ammoniumverbindung homogen gelöst ist.

4. Verfahren nach Anspruch 1,
   **dadurch gekennzeichnet,**
   **dass** die Konzentration der Ammoniumionen oder substituierten Ammoniumionen 500 ppm bis 10 Gew.-% in der Reaktionsmischung beträgt, wobei die Ammoniumverbindung oder substituierte Ammoniumverbindung homogen gelöst ist.

5. Verfahren zur Herstellung von Oximen nach mindestens einem der vorhergehenden Ansprüche,
**dadurch gekennzeichnet,**
**dass** $R_1$, $R_2$, $R_3$ und $R_4$ Wasserstoff bedeutet.

6. Verfahren zur Herstellung von Oximen nach Anspruch 1,
**dadurch gekennzeichnet,**
**dass** als Ammoniumsalz Ammoniumformiat, Ammoniumacetat, Ammoniumpropionat, Ammoniumoxalat oder Ammoniummalonat anwesend ist.

7. Verfahren nach mindestens einem der vorhergehenden Ansprüche,
**dadurch gekennzeichnet,**
**dass** neben der Titan/Silizium/Sauerstoff-Komponente als Cokatalysator mindestens eine weitere Komponente aus einem sauren Feststoff, der ein anorganisches oder organisches Trägermaterial enthält, anwesend ist, wobei entweder das Trägermaterial selbst lewis- oder brönstedsaure Eigenschaften besitzt oder entsprechende lewis- oder brönstedsaure funktionelle Gruppen auf das Trägermaterial aufgebracht werden und die Auftragung solcher Gruppen physikalisch oder chemisch erfolgt.

8. Verfahren nach Anspruch 1,
**dadurch gekennzeichnet,**
**dass** die Titan/Silizium/Sauerstoff-Komponente ein Titansilikalit ist.

9. Verfahren nach Anspruch 1,
**dadurch gekennzeichnet,**
**dass** die Titan/Silizium/Sauerstoff-Komponente der Titansilikalit TS ist.

10. Verfahren nach einem der vorhergehenden Ansprüche,
**dadurch gekennzeichnet,**
**dass** die Ammoniumionen oder substituierten Ammoniumionen in der Reaktionsmischung durch Lösen eines geeigneten Salzes entstehen.

11. Verfahren nach einem der vorhergehenden Ansprüche,
**dadurch gekennzeichnet,**
**dass** die Ammoniumionen in der Reaktionsmischung durch Neutralisation einer freien Säure oder eines geeigneten Derivates davon mit Ammoniak entstehen.

12. Verfahren nach einem der vorhergehenden Ansprüche,
**dadurch gekennzeichnet,**
**dass** als Carbonylverbindung Acetophenon oder cyclische Ketone mit 7 bis 20 Kohlenstoffatomen ammoximiert werden.

13. Verfahren nach einem der vorhergehenden Ansprüche,
**dadurch gekennzeichnet,**
**dass** Cyclododecanon ammoximiert wird.

14. Verfahren nach einem der vorhergehenden Ansprüche,
**dadurch gekennzeichnet,**
**dass** Lösemittel mit ausreichender Löslichkeit für Edukt, Produkt und Ammoniumsalz oder substituiertem Ammoniumsalz zugesetzt werden.

15. Verfahren nach einem der vorhergehenden Ansprüche,
**dadurch gekennzeichnet,**
**dass** als Lösemittel mit Wasser vollständig oder teilweise mischbare Alkohole zugesetzt werden.

16. Verfahren nach Anspruch 14 oder 15,
**dadurch gekennzeichnet,**
**dass** als Lösemittel Methanol, Ethanol oder tert.-Butanol zugesetzt wird.

**17.** Verfahren nach einem der vorhergehenden Ansprüche,
**dadurch gekennzeichnet,**
**dass** die Reaktionstemperatur zwischen 20 °C und 150 °C liegt.

**18.** Verfahren nach einem der vorhergehenden Ansprüche,
**dadurch gekennzeichnet,**
**dass** die Reaktionstemperatur zwischen 60 °C und 100 °C liegt.

**19.** Verfahren nach einem der vorhergehenden Ansprüche,
**dadurch gekennzeichnet,**
**dass** der Druck während der Reaktion 1 bar bis 10 bar beträgt.

**Claims**

**1.** A process for preparing oximes by reacting carbonyl compounds in the liquid phase with hydrogen peroxide and ammonia in the presence of a heterogeneous catalyst based on titanium, silicon and oxygen, **characterized in that** ammonium salts or substituted ammonium salts of the formula $[NR_1R_2R_3R_4]^{\oplus}X^{\ominus}$, where $R_1$, $R_2$, $R_3$ and $R_4$ are each, independently of one another, hydrogen, an aliphatic, unbranched or branched alkyl radical having from 1 to 20 carbon atoms, a cycloaliphatic radical having from 3 to 12 carbon atoms or an aromatic radical having a total of from 6 to 12 carbon atoms and $X^{\ominus}$ is an anion of an acid, are present as a homogeneous solution in the reaction system, the ammonium concentration in the reaction mixture being at least 100 ppm, calculated as $[NR_1R_2R_3R_4]^{\oplus}$, where $X^{\ominus}$ is a halide, nitrate or phosphate ion or an anion of an organic carboxylic acid.

**2.** A process according to claim 1, **characterized in that** the concentration of ammonium ions or substituted ammonium ions is from 100 ppm to 20% by weight in the reaction mixture, the ammonium compound or substituted ammonium compound being homogeneously dissolved.

**3.** A process according to claim 1, **characterized in that** the concentration of ammonium ions or substituted ammonium ions is from 250 ppm to 20% by weight in the reaction mixture, the ammonium compound or substituted ammonium compound being homogeneously dissolved.

**4.** A process according to claim 1, **characterized in that** the concentration of ammonium ions or substituted ammonium ions is from 500 ppm to 10% by weight in the reaction mixture, the ammonium compound or substituted ammonium compound being homogeneously dissolved.

**5.** A process for preparing oximes according to at least one of the preceding claims, **characterized in that** $R_1$, $R_2$, $R_3$ and $R_4$ are each hydrogen.

**6.** A process for preparing oximes according to claim 1, **characterized in that** ammonium formate, ammonium acetate, ammonium propionate, ammonium oxalate or ammonium malonate is present as ammonium salt.

**7.** A process according to at least one of the preceding claims, **characterized in that** , in addition to the titanium/silicon/oxygen component, as cocatalyst, at least one further component comprising an acidic solid which contains an inorganic or organic support material is present, and either the support material itself has Lewis or Bronsted acid properties or appropriate Lewis-acid or Brönsted-acid functional groups are applied to the support material and the application of such groups is effected physically or chemically.

**8.** A process according to claim 1, **characterized in that** the titanium/silicon/oxygen component is a titanium silicalite.

**9.** A process according to claim 1, **characterized in that** the titanium/silicon/oxygen component is the titanium silicalite TS1.

**10.** A process according to any one of the preceding claims, **characterized in that** the ammonium ions or substituted ammonium ions are formed in the reaction mixture by dissolution of a suitable salt.

**11.** A process according to any of the preceding claims, **characterized in that** the ammonium ions are formed in the reaction mixture by neutralization of a free acid or a suitable derivative thereof with ammonia.

**12.** A process according to any one of the preceding claims, **characterized in that** acetophenone or cyclic ketones having from 7 to 20 carbon atoms are ammoximated as carbonyl compound.

**13.** A process according to any one of the preceding claims, **characterized in that** cyclododecanone is ammoximated.

**14.** A process according to any one of the preceding claims, **characterized in that** solvents having sufficient solvent capability for starting material, product and ammonium salt or substituted ammonium salt are added.

**15.** A process according to any one of the preceding claims, **characterized in that** alcohols which are completely or partially miscible with water are added.

**16.** A process according to claim 14 or 15,
**characterized in that** methanol, ethanol or tert-butanol is added as solvent.

**17.** A process according to any one of the preceding claims, **characterized in that** the reaction temperature is from $20°C$ to $150°C$.

**18.** A process according to any one of the preceding claims, **characterized in that** the reaction temperature is from $60°C$ to $100°C$.

**19.** A process according to any one of the preceding claims, **characterized in that** the pressure during the reaction is from 1 bar to 10 bar.

**Revendications**

**1.** Procédé de préparation d'oximes par conversion de composés carbonyles en phase liquide avec du peroxyde d'hydrogène et de l'ammoniac en présence d'un catalyseur hétérogène qui est constitué à base de titane, de silicium et d'oxygène,
**caractérisé en ce que**
des sels d'ammonium ou des sels d'ammonium substitués de formule générale $[NR_1R_2R_3R_4]^+X^-$, dans laquelle $R_1$, $R_2$, $R_3$ et $R_4$ indépendamment les uns des autres représentent un hydrogène, un reste alkyle aliphatique, ramifié ou non ramifié avec 1 à 20 atomes de carbone, un reste cycloaliphatique avec 3 à 12 atomes de carbone ou un reste aromatique avec, au total, 6 à 12 atomes de carbone et $X^-$ représente un anion d'un acide, sont présents, sous forme dissoute et homogène, dans un système réactionnel, la concentration d'ammonium s'élevant dans le mélange réactionnel à au moins 100 ppm, calculée comme $[NR_1R_2R_3R_4]^+$ $X^-$ représentant un ion halogénure, nitrate ou phosphate ou un anion d'un acide carbonique organique.

**2.** Procédé selon la revendication 1,
**caractérisé en ce que**
la concentration des ions ammonium ou des ions ammonium substitué s'élève de 100 ppm à 20 % en poids dans le mélange réactionnel, le composé d'ammonium ou le composé d'ammonium substitué étant dissous de façon homogène.

**3.** Procédé selon la revendication 1,
**caractérisé en ce que**
la concentration des ions ammonium ou des ions ammonium substitué s'élève de 250 ppm à 20 % en poids dans le mélange réactionnel, le composé d'ammonium ou le composé d'ammonium substitué étant dissous de façon homogène.

**4.** Procédé selon la revendication 1,
**caractérisé en ce que**
la concentration des ions ammonium ou des ions ammonium substitué s'élève de 500 ppm à 10 % en poids dans le mélange réactionnel, le composé d'ammonium ou le composé d'ammonium substitué étant dissous de façon homogène.

**5.** Procédé de préparation d'oximes selon au moins l'une quelconque des revendications précédentes,
**caractérisé en ce que**

$R_1$, $R_2$, $R_3$ et $R_4$ représentent un hydrogène.

6. Procédé de préparation d'oximes selon la revendication 1,
   **caractérisé en ce que**
   comme sel d'ammonium sont présents un formiate d'ammonium, un acétate d'ammonium, un propionate d'ammonium, un oxalate d'ammonium ou un malonate d'ammonium.

7. Procédé selon l'une quelconque au moins des revendications précédentes,
   **caractérisé en ce qu'**
   en plus du composant titane/silicium/oxygène, est présent sous la forme d'un cocatalyseur au moins un autre composant obtenu à partir d'une substance solide acide qui contient un substrat inorganique ou organique, le substrat possédant lui-même des propriétés d'acide de Lewis ou de Brönsted ou des groupes fonctionnels d'acides de Lewis ou de Brönsted correspondants étant appliqués sur le substrat et l'application de ces groupes étant réalisée de façon physique ou chimique.

8. Procédé selon la revendication 1,
   **caractérisé en ce que**
   le composant titane/silicium/oxygène est une silicalite de titane.

9. Procédé selon la revendication 1,
   **caractérisé en ce que**
   le composant titane/silicium/oxygène de la silicalite de titane est TS1.

10. Procédé selon l'une quelconque des revendications précédentes,
    **caractérisé en ce que**
    les ions d'ammonium et les ions d'ammonium substitué apparaissent dans le mélange réactionnel par dissolution d'un sel approprié.

11. Procédé selon l'une quelconque des revendications précédentes,
    **caractérisé en ce que**
    les ions d'ammonium apparaissent dans le mélange réactionnel par neutralisation d'un acide libre ou d'un dérivé approprié de celui-ci avec l'ammoniac.

12. Procédé selon l'une quelconque des revendications précédentes,
    **caractérisé en ce que**
    comme composé carbonyle, on ammoxime l'acétophénone ou la cétone cyclique avec 7 à 20 atomes de carbone.

13. Procédé selon l'une quelconque des revendications précédentes,
    **caractérisé en ce que**
    le cyclododécanone est ammoximé.

14. Procédé selon l'une quelconque des revendications précédentes,
    **caractérisé en ce que**
    le solvant est ajouté, avec une solubilité suffisante pour un éduit, au produit et au sel d'ammonium ou au sel d'ammonium substitué.

15. Procédé selon l'une quelconque des revendications précédentes,
    **caractérisé en ce que**
    comme solvant, on ajoute des alcools miscibles partiellement ou totalement à l'eau.

16. Procédé selon la revendication 14 ou 15,
    **caractérisé en ce que**
    comme solvant, on ajoute du méthanol, de l'éthanol ou du tert.-butanol.

17. Procédé selon l'une quelconque des revendications précédentes,
    **caractérise en ce que**
    la température réactionnelle se situe entre 20°C et 150°C.

**18.** Procédé selon l'une quelconque des revendications précédentes, **caractérisé en ce que** la température réactionnelle se situe entre 60°C et 100°C.

**19.** Procédé selon l'une quelconque des revendications précédentes, **caractérisé en ce que** la pression durant la réaction se situe entre 1 bar et 10 bars.